# EUROPÄISCHE PATENTSCHRIFT

(11) **EP 3 510 008 B1**
(45) Veröffentlichungstag und Bekanntmachung des Hinweises auf die Patenterteilung: **04.11.2020**
(21) Anmeldenummer: 17765417.5
(22) Anmeldetag: 08.09.2017
(51) Int. Cl.: C07C 4/16, C07C 4/18, C07C 15/04

(54) **VERFAHREN UND ANLAGE ZUR HERSTELLUNG VON BENZOL**
METHOD AND PLANT FOR PRODUCING BENZENE
PROCÉDÉ ET INSTALLATION DE PRODUCTION DE BENZÈNE

(30) Priorität: 08.09.2016 EP 16187895
(43) Veröffentlichungstag der Anmeldung: 17.07.2019
(73) Patentinhaber: Linde GmbH, 82049 Pullach (DE)
(72) Erfinder: ZIMMERMANN, Heinz, 81476 München (DE); BRUDER, David, 82379 München (DE); HÖFEL, Torben, 81543 München (DE); KURZ, Benedikt, 80686 München (DE); BRUDI, Karlheinz, 85579 Neubiberg (DE); SPINDELNDREHER, Anne, 82377 Penzberg (DE); ANDRE, Matthias, 81475 München (DE); KOLLER, Richard, 81479 München (DE)
(86) Internationale Anmeldenummer: PCT/EP2017/072600
(87) Internationale Veröffentlichungsnummer: WO 2018/046671

(56) Entgegenhaltungen:
- DE-A1- 2 814 367

## Beschreibung

Die Erfindung betrifft ein Verfahren und eine Anlage zur Herstellung von Benzol gemäß den Oberbegriffen der unabhängigen Patentansprüche.

### Stand der Technik

Benzol kann durch Hydrodealkylierung von Verbindungen wie Toluol, Xylolen und alkylierten Aromaten mit neun Kohlenstoffatomen hergestellt werden. Die genannten Ausgangsverbindungen werden häufig in Form einer sogenannten BTX-Fraktion gewonnen, die beispielsweise unter Verwendung von sogenanntem Pyrolysebenzin gebildet werden kann, das beim Dampfspalten anfällt. Alternative Quellen sind das Reformat bei der katalytischen Reformierung sowie das Hydrierbenzin bei der Carbonisierung von Kohle.

Pyrolysebenzin aus der Dampfspaltung umfasst typischerweise überwiegend oder ausschließlich Kohlenwasserstoffe mit fünf bis zehn Kohlenstoffatomen, davon überwiegend Aromaten. Die enthaltenen Aliphaten sind überwiegend ungesättigt und umfassen einen hohen Anteil an Acetylenen und Dienen. Das Pyrolysebenzin ist daher instabil und kann aufgrund der Polymerisationsneigung der genannten Komponenten nicht gelagert werden. Sie wird daher in mehreren Schritten weiterbehandelt. Beispielsweise kann zunächst eine selektive Hydrierung erfolgen, um Acetylene, Diene und Styrole zu Olefinen umzusetzen. Nach Abtrennung von höhermolekularen Komponenten kann das entsprechend behandelte Pyrolysebenzin dann einer Trennung zugeführt werden, in der typischerweise unter anderem eine Fraktion gebildet wird, die überwiegend oder ausschließlich Kohlenwasserstoffe mit sechs bis acht Kohlenstoffatomen enthält. Es handelt sich hierbei um den sogenannten "Herzschnitt" (engl. Heart Cut).

Der Herzschnitt kann einer Hydrodesulfurierung unterworfen werden, in der Olefine zu Paraffinen und Naphthenen und organisch gebundener Schwefel zu Schwefelwasserstoff umgesetzt wird, der in einem nachgeschalteten Stripper ausgetrieben werden kann. Der entsprechend behandelte Herzschnitt kann anschließend einer Aromatenextraktion unterworfen werden, in welcher die BTX-Fraktion von den Aliphaten abgetrennt wird.

Bei der Hydrodealkylierung werden die Alkylreste i.d.R. unter Verbrauch jeweils eines Wasserstoffmoleküls und unter Bildung der entsprechenden Alkane vom Benzolring abgespalten. Es sind katalytische und thermische Hydrodealkylierungsverfahren bekannt. Diesen Verfahren ist gemein, dass zur Hydrodealkylierung jeweils Wasserstoff bereitgestellt werden muss.

Zur Bereitstellung von Wasserstoff für großtechnische Verfahren werden typischerweise Reformierungsverfahren, beispielsweise die katalytische Refomierung in Raffinerien oder die Dampfreformierung, eingesetzt. Bei letzterer werden Kohlenwasserstoffe mit Dampf einem oder mehreren katalytischen Reaktoren zugeführt und dort unter anderem zu Kohlenmonoxid und Wasserstoff, also zu Synthesegas, umgesetzt. Zur Erhöhung des Wasserstoffanteils kommt anschließend typischerweise eine Wassergasshift zum Einsatz, in welcher Kohlenmonoxid mit Wasser zu weiterem Wasserstoff und Kohlendioxid umgesetzt wird. Der Wasserstoff kann anschließend abgetrennt werden.

Zu Details bezüglich der Hydrodealkylierung und der Wasserstoffgewinnung sei auf einschlägige Fachliteratur verwiesen, beispielsweise den Artikel "Benzene" in Ullmann's Encyclopedia of Industrial Chemistry, online seit 15. Juni 2000, DOI 10.1002/14356007.a03_475, insbesondere Abschnitt 5.3.1, "Hydrodealkylation", und den Artikel "Hydrogen" in Ullmann's Encyclopedia of Industrial Chemistry, Onlineausgabe 15. Juni 2000, DOI: 10.1002/14356007.a13_297.

Aus der DE 28 14 367 A1 ist ein Verfahren zur Herstellung von Benzol bekannt, bei dem man eine an Alkylaromaten reiche Kohlenwasserstoff-Charge, die etwa 2 bis 20 Gew.-% Paraffin-Kohlenwasserstoffe (iso und normal) und Naphthene oder Gemische dieser Kohlenwasserstoffe enthält, in einer ersten katalytischen Zone einer Wasserstoffbehandlung bei einer Temperatur von 450 bis 570 °C unter einem Druck von 10 bis 50 bar mit einer Durchflußgeschwindigkeit von 0,1 bis 10 cm³ der flüssigen Charge pro cm³ Katalysator und pro Stunde, bei einem Molverhältnis Wasserstoff/Kohlenwasserstoffe von 2 bis 10 in Gegenwart eines Katalysators auf Mordenit-Basis unterwirft, der weniger als 0,6 Gew.-% Natrium enthält und dessen Porenöffnungen etwa 5 bis 6 Ä betragen und der Kationen der Metalle der Gruppen VIII und IB des Periodensystems enthält, wobei man anschließend mindestens einen Teil des Effluents der ersten katalytischen Zone in eine zweite Zone leitet, wo er einer Hydrodealkylierung unterworfen wird, und zwar entweder thermisch bei 620 bis 720 °C, oder katalytisch auf einem zweiten katalytischen Bett bei einer Temperatur von 500 bis 650 °C unter einem Druck von 10 bis 50 bar, bei ein er Durchflussgeschwindigkeit von 0,1 bis 10 und einem Molverhältnis Wasserstoff/Kohlenwasserstoffe von 2 bis 10, wobei der Katalysator einen nicht-selektiven Träger enthält.

Bei der Gewinnung von Wasserstoff aus Synthesegas für den Einsatz in der Hydrodealkylierung wird typischerweise eine Tieftemperaturtrennung eingesetzt, die der Hydrodesulfurierung vorgeschaltet ist. In der Hydrodesulfurierung nicht umgesetzter Wasserstoff, der erneut in der Hydrodesulfurierung eingesetzt werden soll, muss ferner herkömmlicherweise aufwendig von Spuren von Benzol befreit werden. Da bei der Hydrodealkylierung typischerweise Schwefelverbindungen zugesetzt werden, um die Verkokung der Reaktoren zu verhindern, ist schließlich eine Laugenwäsche erforderlich. Daneben wird in den Hydrodealkylierungen eine Benzolabsorbersäule eingesetzt, die die Aufgabe hat, die Benzolverluste mit dem Leichtgasstrom zu minimieren.

Die genannten Faktoren erhöhen die Kosten und den apparativen Aufwand bei der Herstellung von Benzol durch Hydrodealkylierung. Die vorliegende Erfindung stellt sich die Aufgabe, hier Verbesserungen zu schaffen.

### Offenbarung der Erfindung

Vor diesem Hintergrund schlägt die vorliegende Erfindung ein Verfahren und eine Anlage zur Herstellung von Benzol mit den Merkmalen der unabhängigen Patentansprüche vor. Ausgestaltungen sind jeweils Gegenstand der abhängigen Patentansprüche sowie der nachfolgenden Beschreibung.

Vor der Erläuterung der Merkmale und Vorteile der vorliegenden Erfindung werden deren Grundlagen und die verwendeten Begriffe erläutert.

Flüssige und gasförmige Gemische können im hier verwendeten Sprachgebrauch reich oder arm an einer oder mehreren Komponenten sein, wobei "reich" für einen Gehalt von wenigstens 50%, 75%, 90%, 95%, 99%, 99,5%, 99,9% oder 99,99% und "arm" für einen Gehalt von höchstens 50%, 25%, 10%, 5%, 1%, 0,1% oder 0,01% auf molarer, Gewichts- oder Volumenbasis stehen kann. Der Begriff "überwiegend" kann der Definition von "reich" entsprechen. Flüssige und gasförmige Gemische können im hier verwendeten Sprachgebrauch ferner angereichert oder abgereichert an einer oder mehreren Komponenten sein, wobei sich diese Begriffe auf einen entsprechenden Gehalt in einem Ausgangsgemisch beziehen, aus dem der flüssige oder gasförmige Strom erhalten wurde. Das flüssige oder gasförmige Gemisch ist "angereichert", wenn es zumindest den 1,1-fachen, 1,5-fachen, 2-fachen, 5-fachen, 10-fachen, 100-fachen oder 1.000-fachen Gehalt, "abgereichert", wenn es höchstens den 0,9-fachen, 0,5-fachen, 0,1-fachen, 0,01-fachen oder 0,001-fachen Gehalt einer entsprechenden Komponente, bezogen auf das Ausgangsgemisch, enthält. Ist vorliegend beispielsweise von "Methan" oder "Wasserstoff" bzw. einer entsprechenden Fraktion die Rede, sei darunter auch ein Gemisch verstanden, das reich an der entsprechenden Komponente ist. Es kann sich jedoch auch um das jeweilige Reingas handeln.

Ein flüssiges oder gasförmiges Gemisch ist von einem anderen flüssigen oder gasförmigen Gemisch (auch als Ausgangsgemisch bezeichnet) "abgeleitet" oder aus diesem Gemisch oder unter Verwendung dieses Gemischs "gebildet", wenn es zumindest einige in dem Ausgangsstrom enthaltene oder aus diesem erhaltene Komponenten aufweist. Ein in diesem Sinne gebildetes Gemisch kann aus dem Ausgangsgemisch durch Abtrennen oder Abzweigen eines Teilstroms oder einer oder mehrerer Komponenten, Anreichern oder Abreichern bezüglich einer oder mehrerer Komponenten, chemisches oder physikalisches Umsetzen einer oder mehrerer Komponenten, Erwärmen, Abkühlen, Druckbeaufschlagen und dergleichen gebildet werden. Ein "Bilden", beispielsweise eines Einsatzgemischs für einen anschließenden Trennprozess, kann jedoch auch einfach das Führen eines entsprechenden Gemischs in einer geeigneten Leitung und ein Zuführen zu dem Trennprozess darstellen.

### Vorteile der Erfindung

Die vorliegende Erfindung beruht auf der Erkenntnis, dass eine weitgehende Integration einer Hydrodealkylierung und eines Dampfspaltverfahrens besondere Vorteile bietet und die eingangs erwähnten Nachteile zumindest zum Teil überwindet. Verfahren und entsprechende Anlagen zur Gewinnung von Olefinen wie Ethylen durch Dampfspalten (engl. Steam Cracking) sind bekannt und z.B. im Artikel "Ethylene" in Ullmann's Encyclopedia of Industrial Chemistry, online seit 15. April 2009, DOI 10.1002/14356007.a10_045.pub3, beschrieben.

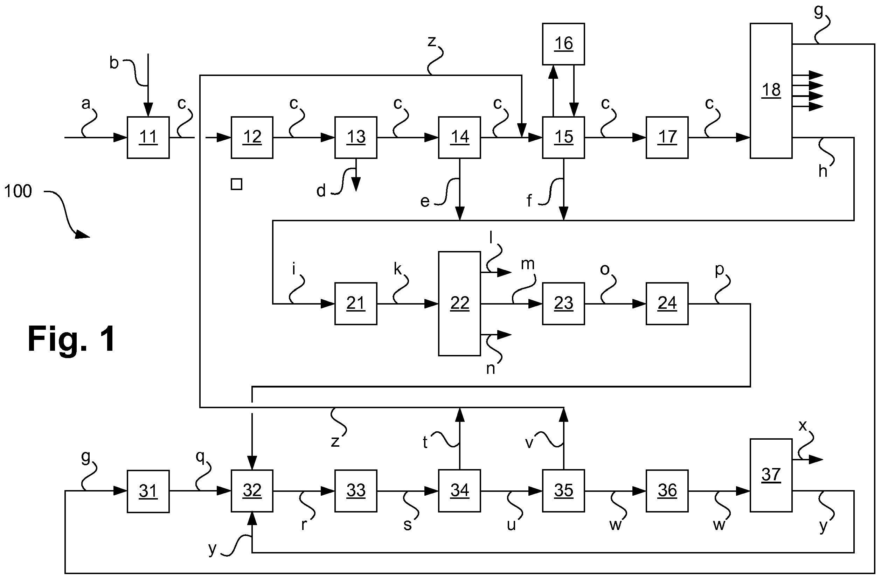

Beim Dampfspalten werden bekanntermaßen Stoffgemische gebildet, die einer geeigneten Aufbereitung, und, zur Trennung in Komponenten oder Komponentengruppen, bekannten Trennsequenzen unterworfen werden können. Ein Beispiel für eine derartige Trennsequenz ist unter Bezugnahme auf die beigefügte Figur 1 näher erläutert. Entsprechende Trennsequenzen sind aus dem zitierten Stand der Technik bekannt und unterscheiden sich im Wesentlichen durch die Reihenfolge der verwendeten Trennschritte.

Typischerweise wird ein entsprechendes Stoffgemisch, nachfolgend auch als "Produktgemisch" des Dampfspaltens bezeichnet, obgleich ein derartiges Stoffgemisch nicht nur die gewünschten Produkte, sondern auch Nebenprodukte und nicht umgesetzte Edukte enthalten kann, zunächst einer Kühlung, beispielsweise in einem Linearkühler (engl. Transfer Line Exchanger, TLE) unterworfen. Anschließend erfolgt eine Abtrennung schwererer Komponenten, typischerweise unter Verwendung eines Öl- und eines Wasserkreislaufs. Hierbei kann eine Pyrolysebenzinfraktion gebildet und Wasser rückgewonnen werden. Weiteres Pyrolysebenzin scheidet sich in einer sich anschließenden Verdichtung aus dem Produktgemisch ab. Im Zuge der Verdichtung, d.h. insbesondere auf einer Zwischenstufe eines verwendeten mehrstufigen Verdichters, erfolgt typischerweise eine Sauergasentfernung, typischerweise unter Verwendung einer Amin- und/oder Laugenwäsche. Das entsprechend aufbereitete Produktgemisch wird anschließend getrocknet und vorgekühlt, bevor es einer Tieftemperaturtrennung unterworfen wird.

Die Tieftemperaturtrennung kann beispielsweise in Form eines sogenannten "Deethanizer First"-, eines "Demethanizer First"- oder eines "Depropanizer First"-Verfahren ausgestaltet sein. Zu entsprechenden Details sei auf den eingangs zitierten Stand der Technik verwiesen. In einer entsprechenden Tieftemperaturtrennung werden typischerweise eine Wasserstofffraktion sowie eine Methanfraktion getrennt. Die Wasserstofffraktion weist typischerweise einen Wasserstoffgehalt von 80% - 95% auf.

Zur Bildung einer entsprechenden Wasserstofffraktion kann beispielsweise auch zunächst eine überwiegend oder ausschließlich Wasserstoff und Methan enthaltende Fraktion gebildet werden, aus welcher der Wasserstoff anschließend beispielsweise unter Verwendung einer Druckwechseladsorption abgetrennt werden kann. Die Wasserstofffraktion wird typischerweise unter Verwendung eines bekannten Demethanizers gebildet, in dem schwerere Komponenten flüssig aus einem entsprechenden Gasgemisch abgeschieden werden.

Weitere Fraktionen, die in einer entsprechenden Tieftemperaturtrennung gebildet werden können, sind beispielsweise eine Fraktion, die überwiegend oder ausschließlich Kohlenwasserstoffe mit zwei Kohlenstoffatomen enthält, insbesondere Ethan und Ethylen. Hieraus kann Ethylenfraktion abgetrennt und als Produkt bereitgestellt werden. Das Ethan kann beispielsweise zum Dampfspalten zurückgeführt werden. Bezüglich der Gewinnung weiterer Fraktionen sei auf den Stand der Technik verwiesen. Einer entsprechenden Tieftemperaturtrennung bzw. einzelnen Schritten dieser kann insbesondere eine Hydrierung vor-, zwischen- oder nachgeschaltet sein. Diese dient insbesondere dazu, in dem Produktgemisch enthaltene Acetylene zu den entsprechenden Olefinen umzusetzen. In einer entsprechenden Tieftemperaturtrennung kann insbesondere auch eine weitere Pyrolysebenzinfraktion gebildet werden.

Die vorliegende Erfindung schlägt nun vor, die Wasserstofffraktion aus der Tieftemperaturtrennung der Dampfspaltung anstelle einer separat bereitgestellten Wasserstofffraktion in der Hydrodealkylierung einzusetzen. Auf diese Weise kann auf die Bereitstellung eines separaten Reformierungsverfahrens und der entsprechenden Tieftemperaturtrennschritte und die Absorbersäule verzichtet werden. Die Wasserstofffraktion aus der Tieftemperaturtrennung des Dampfspaltverfahrens erweist sich als ausreichend rein und liegt in geeignetem Zustand vor, um direkt in der Hydrodealkylierung eingesetzt zu werden. Vorzugsweise ist lediglich eine Verdichtung dieser Wasserstofffraktion erforderlich, um sie einem entsprechenden Reaktor zuführen zu können.

Ein weiterer Aspekt der vorliegenden Erfindung ist die Behandlung des in der Hydrodealkylierung nicht umgesetzten Wasserstoffs beziehungsweise einer entsprechenden Leichtgasfraktion. Solcher Wasserstoff wird zusammen mit den bei der Dealkylierung gebildeten kurzkettigen Alkanen aus einem Produktgemisch der Hydrodealkylierung in einer entsprechenden, diese Komponenten enthaltenden, Leichtgasfraktion abgetrennt. Die Leichtgasfraktion umfasst damit neben Wasserstoff auch kurzkettige Alkane, insbesondere Methan, optional Ethan und/oder Ethlyen, und weiter optional Propan und/oder Propylen.

Herkömmlicherweise muss entsprechender Wasserstoff aufwendig aufgereinigt werden, um erneut in der Hydrodealkylierung eingesetzt werden zu können. Die vorliegende Erfindung schlägt im Gegensatz dazu nun vor, diese Fraktion der Trennung zuzuführen, der auch das Produktgemisch des Dampfspaltverfahrens unterworfen wird. Insbesondere wird eine entsprechende Fraktion dabei stromauf einer Verdichtung in einer entsprechenden Trennsequenz zugeführt. Wie erwähnt, erfolgt im Zuge einer derartigen Verdichtung typischerweise auch eine Amin- oder Laugenwäsche, in der Sauergase aus einem entsprechenden Gasgemisch ausgewaschen werden. Allgemeiner erfolgt im Zuge der Trennung, der das Produktgemisch des Dampfspaltverfahrens unterworfen wird, eine Sauergasentfernung. Dabei wird die genannte Leichtgasfraktion vorteilhafterweise nicht nur stromauf der Verdichtung sondern erfindungsgemäß auch der Sauergasentfernung, beispielsweise einer Amin- oder Laugenwäsche, der Trennung zugeführt. Da, wie erwähnt, bei der Hydrodealkylierung auch Schwefelverbindungen eingesetzt werden, und da diese in ein entsprechendes Produktgemisch und damit auch in die Leichtgasfraktion übergehen können, kann auf diese Weise eine Auswaschung bzw., je nach dem eingesetzten Verfahren anderweitige, Entfernung von Schwefelverbindungen erfolgen, ohne dass eine separate Laugenwäsche oder ein anderes separates Verfahren zur Sauergasentfernung erforderlich wäre.

Insgesamt schlägt die vorliegende Erfindung ein Verfahren zur Herstellung von Benzol vor, bei dem ein erstes Einsatzgemisch gebildet wird, das alkylierte Aromaten und Wasserstoff enthält, und bei die der in dem ersten Einsatzgemisch enthaltenen alkylierten Aromaten und der in dem ersten Einsatzgemisch enthaltene Wasserstoff durch Hydrodealkylierung unter Erhalt eines ersten Produktgemisches teilweise zu Benzol umgesetzt werden. Das erste Produktgemisch enthält das Benzol, die nicht umgesetzten alkylieren Aromaten, bei der Umsetzung der alkylierten Aromaten zu den Benzol gebildete Alkane mit einem bis drei Kohlenstoffatomen und den nicht umgesetzten Wasserstoff. Wie insoweit aus dem Stand der Technik bekannt, wird zumindest ein Teil der Alkane mit einem bis drei Kohlenstoffatomen und des Wasserstoffs unter Erhalt einer Leichtgasfraktion aus dem ersten Produktgemisch abgetrennt. Die Leichtgasfraktion enthält insbesondere Wasserstoff und die genannten kurzkettigen Alkane, kann aber auch Spuren von Benzol enthalten.

Die vorliegende Erfindung schlägt nun, wie bereits mit anderen Worten erläutert, vor, dass der in dem ersten Einsatzgemisch enthaltene Wasserstoff zumindest zum Teil unter Verwendung einer Tieftemperaturtrennung bereitgestellt wird, welcher zumindest einem Teil eines zweiten Produktgemischs zugeführt wird, wobei das zweite Produktgemisch zumindest zum Teil durch Dampfspalten eines zweiten Einsatzgemischs gebildet wird, und dass die Leichtgasfraktion ebenfalls zumindest zum Teil der Tieftemperaturtrennung zugeführt wird. Durch das erfindungsgemäße Verfahren werden Synergieeffekte zwischen einem Dampfspaltverfahren und einer Hydrodealkylierung genutzt, die insbesondere einen Verzicht auf eine separate Bereitstellung von Wasserstoff für die Hydrodealkylierung und eine gesonderte Behandlung einer entsprechenden Leichtgasfraktion ermöglichen. Details und Vorteile wurden bereits zuvor erläutert.

Wie ebenfalls bereits erwähnt, wird das zweite Produktgemisch oder dessen der Tieftemperaturtrennung zugeführte Teil und die Leichtgasfraktion oder deren der Tieftemperaturtrennung zugeführter Teil verdichtet und anschließend der Tieftemperaturtrennung unterworfen. Wie erwähnt, wird im Zuge der Verdichtung eine Sauergasentfernung vorgenommen, die insbesondere auch für die Behandlung der Leichtgasfraktion aus der Hydrodealkylierung vorteilhaft ist. Eine Sauergasentfernung erfolgt "im Zuge der Verdichtung", weil sie insbesondere auf einem Zwischendruck, d.h. nach einer oder mehreren ersten und vor einem oder mehreren zweiten Verdichtungsstufen bzw. Verdichtungsschritten durchgeführt wird. Die Sauergasentfernung kann im Rahmen der vorliegenden Erfindung insbesondere eine Laugen- und/oder Aminwäsche umfassen.

Mit anderen Worten ist die Tieftemperaturtrennung Teil einer Trennsequenz bzw. Bearbeitungssequenz, wobei in dieser Trennsequenz bzw. Bearbeitungssequenz vor der Tieftemperaturtrennung auch eine Verdichtung erfolgen kann und erfindungsgemäß eine Sauergasentfernung vorgenommen wird. Die Sauergasentfernung muss dabei nicht im Zuge der Verdichtung erfolgen, wie sie zuvor erläutert wurde. In jedem Fall kann in besonders vorteilhafter Weise eine Synergie dadurch erzielt werden, dass die Leichtgasfraktion stromauf der Verdichtung und/oder der Sauergasentfernung der Trennsequenz bzw. Bearbeitungssequenz zugeführt wird. Auf diese Weise wird erfindungsgemäß auf eine separate Sauergasentfernung verzichtet.

Grundsätzlich kann der Wasserstoff in der Tieftemperaturtrennung auf beliebige Weise abgetrennt werden. In dem Verfahren ist es jedoch vorteilhaft, wenn die Tieftemperaturtrennung eine Demethanisierung umfasst, und wenn der in dem ersten Einsatzgemisch enthaltene Wasserstoff zumindest zum Teil unter Verwendung der Demethanisierung bereitgestellt wird. Eine entsprechende Demethanisierung kann als erster ("Demethanizer First"), zweiter ("Deethanizer First") oder auch als weiterer Schritt in einer entsprechenden Trennsequenz erfolgen.

Es ist von besonderem Vorteil, wenn ferner unter Verwendung zumindest eines Teils des zweiten Produktgemischs, also des Produktgemischs des Dampfspaltverfahrens, eine Pyrolysebenzinfraktion bereitgestellt wird. Wie erwähnt, kann eine entsprechende Pyrolysebenzinfraktion insbesondere in einer Wasserwäsche anfallen, der ein entsprechendes Produktgemisch unterworfen wird. Weiteres Pyrolysebenzin kann bei der Verdichtung und in der Tieftemperaturtrennung gebildet werden. Die vorliegende Erfindung schlägt nun vor, eine entsprechende Pyrolysebenzinfraktion ebenfalls zur Gewinnung von Benzol einzusetzen.

Besonders vorteilhaft ist es hierbei, wenn zumindest ein Teil der in dem ersten Einsatzgemisch enthaltenen alkylierten Aromaten durch eine Aufbereitung zumindest eines Teils der Pyrolysebenzinfraktion bereitgestellt wird. Auf diese Weise lässt sich eine weitere stoffliche Integration zwischen dem Dampfspaltverfahren und der Hydrodealkylierung schaffen.

Besonders vorteilhaft ist es dabei, wenn die Aufbereitung der Pyrolysebenzinfraktion oder deren Teil eine Hydrierung und/oder Trennung und/oder Hydrodesulfurierung und/oder Aromatenextraktion umfasst. Entsprechende Schritte werden vom Fachmann je nach Bedarf und insbesondere auch je nach Zusammensetzung einer entsprechenden Pyrolysebenzinfraktion gewählt. Zu Details sei beispielsweise auf die Erläuterungen zur beigefügten Figur 1 und auch Fachliteratur verwiesen. Insbesondere kann im Rahmen der vorliegenden Erfindung beispielsweise das erste Einsatzgemisch derart gebildet werden, dass es im oben erläuterten Sinn arm an oder frei von nichtaromatischen Verbindungen ist. Dies kann beispielsweise dadurch erzielt werden, dass das erste Einsatzgemisch zumindest teilweise aus einer oder mehreren Fraktionen aus einer Aromatenextraktion gebildet wird. Auf diese Weise kann verhindert werden, dass nennenswerte Mengen an Verbindungen, die bekanntermaßen bei ihrer Anwesenheit die Exothermizität der Hydrodealkylierung in übermäßiger Weise steigern können, in die Hydrodealkylierung gelangen. Auf diese Weise wird eine Verwendung gesonderter Verfahrensschritte vermieden.

Wie bereits erwähnt, lässt sich im Rahmen der vorliegenden Erfindung insbesondere eine separate Bereitstellung von Wasserstoff für die Hydrodealkylierung einsparen. Jedoch wird der in der Tieftemperaturtrennung bereitgestellte und in dem ersten Einsatzgemisch enthaltene Wasserstoff zur Bildung des ersten Einsatzgemischs vorteilhafterweise einer Verdichtung unterworfen, damit dieser Wasserstoff auch einem für die Hydrodealkylierung geeigneten Druck vorliegt.

Insbesondere eignet sich das Verfahren der vorliegenden Erfindung für Dampfspaltverfahren, denen Flüssigeinsätze unterworfen werden, insbesondere enthält daher das zweite Einsatzgemisch Naphtha. Die alkylierten Aromaten umfassen vorteilhafterweise Toluol und Xylole.

Stromab der Hydrodealkylierung wird ferner zumindest ein Teil des Benzols und der nicht umgesetzten alkylierten Aromaten unter Erhalt einer Flüssigfraktion aus dem ersten Produktgemisch abgetrennt. Unter Verwendung zumindest eines Teils dieser Flüssigfraktion wird schließlich eine überwiegend oder ausschließlich Benzol enthaltende Fraktion und eine überwiegend oder ausschließlich nicht umgesetzte alkylierte Aromaten enthaltene Fraktion gebildet. Erstere kann als Produktfraktion ausgeführt werden, letztere kann insbesondere zumindest zum Teil die Hydrodealkylierung zurückgeführt werden.

Die vorliegende Erfindung erstreckt sich ferner auf eine Anlage zur Herstellung von Benzol, bezüglich derer auf den entsprechenden Patentanspruch verwiesen wird. Eine entsprechende Anlage ist insbesondere zur Durchführung eines Verfahrens eingerichtet, wie es zuvor erläutert wurde, und weist hierzu entsprechend ausgebildete Mittel auf. Bezüglich Merkmalen und Vorteilen einer entsprechenden Anlage sei daher auf die obigen Erläuterungen bezüglich des erfindungsgemäßen Verfahrens und seiner Ausgestaltungen verwiesen.

Die Erfindung wird nachfolgend unter Bezugnahme auf die beigefügte Zeichnung näher erläutert, welche eine Ausgestaltung eines erfindungsgemäßen Verfahrens veranschaulicht.

### Kurze Beschreibung der Zeichnung

Figur 1 veranschaulicht ein Verfahren gemäß einer Ausführungsform einer Erfindung in Form eines schematischen Ablaufplans.

### Ausführliche Beschreibung der Zeichnung

Figur 1 veranschaulicht ein Verfahren gemäß einer besonders bevorzugten Ausführungsform der Erfindung. Das Verfahren ist insgesamt mit 100 bezeichnet. Werden nachfolgend Verfahrensmerkmale bzw. Verfahrensschritte erläutert, betreffen diese Erläuterungen gleicherweise in einer entsprechenden Anlage vorgesehene Elemente. Wird daher nachfolgend das Verfahren beschrieben, gelten die entsprechenden Erläuterungen für eine entsprechende Anlage in gleicher Weise.

In dem in Figur 1 veranschaulichten Verfahren 100 werden ein Kohlenwasserstoffstrom a und ein Dampfstrom b einem Dampfspaltofen 11 zugeführt. Dabei wird ein Spaltgas gebildet und in Form eines Spaltgasstroms c aus dem Dampfspaltofen 11 ausgeleitet. Die Darstellung ist hier insbesondere insoweit stark vereinfacht, als in der Praxis mehrere Einsatzströme und/oder Dampfströme sowie zusätzlich rückgeführte Stoffströme und dergleichen verwendet werden können, die einem oder mehreren Dampfspaltöfen, die unter gleichen oder unterschiedlichen Bedingungen betrieben werden können, zugeführt werden können. Beispielsweise können auch ein oder mehrere für (vollständig, überwiegend oder teilweise) flüssige Einsatzströme ausgelegte Dampfspaltöfen und/oder ein oder mehrere für (vollständig, überwiegend oder teilweise) gasförmige Einsatzströme ausgelegte Dampfspaltöfen vorgesehen sein. Entsprechend können auch mehrere Spaltgasströme gebildet werden, die beispielsweise vereinigt werden können. Auch die nachfolgend erläuterten Stoffströme und Anlagenteile können ein- oder mehrfach vorhanden sein.

Der Spaltgasstrom c wird im dargestellten Beispiel einer Kühlung 12, beispielsweise unter Verwendung eines Linearkühlers, zugeführt. In einer Primärfraktionierung 13 werden, beispielsweise unter Verwendung eines Ölkreislaufs oder mittels anderer aus dem Stand der Technik bekannter Verfahren, schwere Komponenten mit einem Siedepunkt von typischerweise mehr als 200 °C aus d em Spaltgas abgeschieden und im dargestellten Beispiel in Form eines Pyrolyseölstroms d abgezogen. Das entsprechend von schweren Komponenten befreite Spaltgas wird in Form des weiterhin mit c bezeichneten Spaltgasstroms einer Wasserwäsche 13 zugeführt, wo es unter Verwendung von Waschwasser von Komponenten der Pyrolysebenzinfraktion befreit und vom bei der Spaltung verwendenten Dampfes durch Kondensation abgetrennt wird. Diese Kohlenwasserstoffkomponenten können auch beispielsweise in die Primärfraktionierung 13 zurückgeführt und dort zum Auswaschen der schweren Komponenten verwendet werden. Zumindest ein Teil der Pyrolysebenzinfraktion wird im dargestellten Beispiel in Form eines Pyrolysebenzinstroms e abgezogen.

Auch wenn dem Spaltgasstrom im dargestellten Beispiel nun ein Stoffstrom z (siehe unten) zugespeist wird, ist ein entsprechender Sammelstrom hier weiterhin mit c bezeichnet. Dieser wird einer Verdichtung 15 zugeführt, der eine Sauergasentfernung 16 zugeordnet ist. Die Verdichtung 15 erfolgt über mehrere Stufen, auf eine Zwischenstufe wird der verdichtete Stoffstrom dabei zur Sauergasentfernung 16 geführt. Auch andere Konfigurationen sind möglich. Bei der Verdichtung scheiden sich weitere Komponenten der Pyrolysebenzinfraktion ab, die in Form eines weiteren Pyrolysebenzinstroms f abgezogen werden. Das von Sauergasen befreite Gasgemisch wird in Form eines weiterhin mit c bezeichneten Stoffstroms nun einer Vorkühlung und Trocknung 17 zugeführt und dort von Restwasser befreit und vorgekühlt, bevor es in eine Tieftemperaturtrennung 18 eingespeist wird. Zu Details der Tieftemperaturtrennung 18 sei auf die eingangs zitierte Fachliteratur verwiesen. Die Tieftemperaturtrennung 18 ist hier nur der Übersichtlichkeit halber in Form einer einzelnen Einheit veranschaulicht. In der Praxis sind in einer entsprechenden Tieftemperaturtrennung 18 sequentiell angeordnete Trenneinheiten (beispielsweise Deethanizer, Demethanizer, Depropanizer usw.) vorgesehen.

In der Tieftemperaturtrennung 18 wird aus dem Spaltgas eine Reihe von Fraktionen gebildet, von denen im vorliegenden Fall nur eine Wasserstofffraktion und eine weitere Pyrolysebenzinfraktion von Interesse sind und daher näher erläutert werden. Diese werden in Form eines Wasserstoffstroms g und eines weiteren Pyrolysebenzinstroms h aus der Tieftemperaturtrennung 18 ausgeführt. Die Wasserstofffraktion kann beispielsweise aus einem überwiegend oder ausschließlich Wasserstoff und Methan enthaltenden Gasgemisch, das in der Tieftemperaturtrennung in einem Demethanizer gebildet wird, abgetrennt werden. Sie enthält beispielsweise, wie erwähnt, 90% Wasserstoff. Die Pyrolysebenzinfraktion wird beispielsweise in einem Debutanizer gebildet, in dem Kohlenwasserstoffe mit vier Kohlenstoffatomen aus einem diese Kohlenwasserstoffe mit vier Kohlenstoffatomen sowie schwerere Kohlenwasserstoffe enthaltenden Stoffgemisch abgetrennt werden. Die in dem Debutanizer gebildete Pyrolysebenzinfraktion enthält daher die genannten schwereren Kohlenwasserstoffe, insbesondere Kohlenwasserstoffe mit fünf bis zehn Kohlenstoffatomen.

Weitere in der Tieftemperaturtrennung 18 gebildete Fraktionen, die hier nicht separat erläutert werden, umfassen beispielsweise eine Fraktion, die überwiegend oder ausschließlich Methan enthält, eine Fraktion, die überwiegend oder ausschließlich Kohlenwasserstoffe mit zwei Kohlenstoffatomen enthält, eine Fraktion, die überwiegend oder ausschließlich Kohlenwasserstoffe mit drei Kohlenstoffatomen enthält und eine Fraktion, die überwiegend oder ausschließlich Kohlenwasserstoffe mit zwei Kohlenstoffatomen enthält. Es können auch Teilfraktionen entsprechender Fraktionen gebildet werden, beispielsweise kann aus der Fraktion, die überwiegend oder ausschließlich Kohlenwasserstoffe mit zwei Kohlenstoffatomen enthält, eine Fraktion, die überwiegend oder ausschließlich Ethylen enthält und eine Fraktion, die überwiegend oder ausschließlich Ethan enthält, gebildet werden. Letztere kann beispielsweise in den Spaltofen 11 oder einen von mehreren solcher Spaltöfen, insbesondere einen separaten, für gasförmige Einsätze ausgelegten Spaltofen, zurückgeführt werden. Entsprechendes gilt auch für die weiteren Fraktionen. Alle Fraktionen können geeigneten Nachbehandlungs-, Trenn-, Umsetzungs- und Aufbereitungsschritten unterworfen werden. Die Tieftemperaturtrennung 18 kann beispielsweise auch Hydrierschritte umfassen oder solche Hydrierschritte können der Tieftemperaturtrennung 18 vorgeschaltet und/oder nachgeschaltet sein.

Die Pyrolysebenzinströme e, f und h werden im Rahmen der hier veranschaulichten Ausführungsform der Erfindung zu einem Pyrolysebenzinsammelstrom i vereinigt, können aber auch separat verwendet werden. Die Pyrolysebenzinfraktion umfasst überwiegend oder ausschließlich Kohlenwasserstoffe mit fünf bis zehn Kohlenstoffatomen, davon überwiegend Aromaten. Die enthaltenen Aliphaten sind überwiegend ungesättigt und umfassen einen hohen Anteil an Acetylenen und Dienen. Die Pyrolysebenzinfraktion ist daher instabil und kann aufgrund der Polymerisationsneigung der genannten Komponenten nicht gelagert werden. In Abhängigkeit von den nachgeordneten Verfahrensschritten kann die Pyrolysebenzinfraktion daher in mehreren Schritten weiterbehandelt werden. Am gängigsten, und hier veranschaulicht, ist dabei die selektive Hydrierung 21 der gesamten Pyrolysebenzinfraktion, um Acetylene, Diene und Styrole zu Olefinen umzusetzen. Nach Abtrennung von höhermolekularen Komponenten (nicht veranschaulicht) kann die entsprechend behandelte Pyrolysebenzinfraktion in Form eines Stoffstroms k einer Trennung 22 zugeführt werden.

In der Trennung 22 werden im dargestellten Beispiel drei Fraktionen gebildet und in Form entsprechender Stoffströme abgezogen. Es handelt sich um eine Fraktion, die überwiegend oder ausschließlich Kohlenwasserstoffe mit fünf Kohlenstoffatomen enthält (Stoffstrom I), eine Fraktion, die überwiegend oder ausschließlich Kohlenwasserstoffe mit sechs bis acht Kohlenstoffatomen enthält (sogenannter Herzschnitt, engl. Heart Cut, Stoffstrom m), und eine Fraktion, die überwiegend oder ausschließlich schwerere Kohlenwasserstoffe enthält (Stoffstrom n). Der Herzschnitt kann einer Hydrodesulfurierung 23 unterworfen werden, in der Olefine zu Paraffinen und Naphthenen und gebundener Schwefel zu Schwefelwasserstoff umgesetzt wird, der in einem nachgeschalteten Stripper ausgetrieben werden kann (nicht veranschaulicht). Der entsprechend behandelte Herzschnitt wird in Form eines Stoffstroms o einer Aromatenextraktion 24 unterworfen, in welcher in an sich bekannter Weise Aromaten (die bereits erwähnte BTX-Fraktion) von Aliphaten getrennt werden. Aus der Aromatenextraktion 24 wird im dargestellten Beispiel ein Aromatenstrom p ausgeführt, die Aliphaten sind nicht dargestellt.

Der Aromatenstrom p wird zusammen mit dem in einem Wasserstoffverdichter 31 verdichteten Wasserstoffstrom g, hier mit q bezeichnet, bei Bedarf einer Aufbereitung 32, die beispielsweise eine Erwärmung und ggf. eine Hydrierung umfassen kann, und anschließend in Form eines Einsatzstroms r einer Hydrodealkylierung 33 zugeführt. Ein in der Hydrodealkylierung 33 gebildetes Produktgemisch wird abgekühlt (nicht gezeigt) und in Form eines Produktstroms s einer Phasentrennung 34 zugeführt. In der Phasentrennung 34 wird unter Verbleib einer Gasfraktion eine flüssige Fraktion abgeschieden. Die Gasfraktion, die überwiegend oder ausschließlich die in der Hydrodealkylierung 33 von den alkylierten Aromaten abgespaltenen Alkane, Restwasserstoff und Spuren an Aromaten enthält, wird in Form eins Stoffstroms t abgezogen. Die flüssige Fraktion, die überwiegend Aromaten enthält, wird in Form eines Stoffstroms u in eine Stabilisierung 35 überführt, in der verbliebene Reste von Wasserstoff und Alkanen ausgetrieben werden. Die ausgetriebene Fraktion wird gasförmig in Form eines Stoffstroms v abgezogen.

Es verbleibt eine flüssige Fraktion, die in Form eines Stoffstroms w beispielsweise einer Lehmbehandlung 26 (engl: clay treatment) und anschließend einer Trennung 37 zugeführt werden kann. In der Trennung 37 kann eine Fraktion, die überwiegend oder ausschließlich dealkylierte Aromaten enthält, in Form eines Stoffstroms x abgezogen werden. Nicht dealkylierte Aromaten können in Form eines überwiegend oder ausschließlich solche Aromaten enthaltenden Stoffstroms y in die Aufbereitung 32 oder die Hydrodealkylierung 33 zurückgeführt werden. Die Stoffströme t und v werden im dargestellten Beispiel zu einem Sammelstrom z vereinigt, der stromauf oder in der Verdichtung 15 mit dem Spaltgasstrom c vereinigt werden kann.

## Patentansprüche

1. Verfahren (100) zur Herstellung von Benzol, bei dem ein erstes Einsatzgemisch gebildet wird, das alkylierte Aromaten und Wasserstoff enthält, und bei dem die in dem ersten Einsatzgemisch enthaltenen alkylierten Aromaten mit dem in dem ersten Einsatzgemisch enthaltenen Wasserstoff durch Hydrodealkylierung (33) unter Erhalt eines ersten Produktgemischs teilweise zu dem Benzol umgesetzt werden, wobei das erste Produktgemisch das Benzol, die nicht umgesetzten alkylierte Aromaten, bei der Umsetzung der alkylierten Aromaten zu dem Benzol gebildete Alkane mit einem bis drei Kohlenstoffatomen und den nicht umgesetzten Wasserstoff enthält, und wobei zumindest ein Teil der Alkane mit einem bis drei Kohlenstoffatomen und des Wasserstoffs unter Erhalt einer Leichtgasfraktion aus dem ersten Produktgemisch abgetrennt werden, **dadurch gekennzeichnet, dass** der in dem ersten Einsatzgemisch enthaltene Wasserstoff zumindest zum Teil unter Verwendung einer Tieftemperaturtrennung (18) bereitgestellt wird, welcher zumindest ein Teil eines zweiten Produktgemischs zugeführt wird, wobei das zweite Produktgemisch zumindest zum Teil durch Dampfspalten (11) eines zweiten Einsatzgemischs gebildet wird, und dass die Leichtgasfraktion ebenfalls zumindest zum Teil der Tieftemperaturtrennung (18) zugeführt wird, wobei das zweite Produktgemisch oder dessen der Tieftemperaturtrennung (18) zugeführter Teil und die Leichtgasfraktion oder deren der Tieftemperaturtrennung (18) zugeführter Teil einer Sauergasentfernung (16) und anschließend der Tieftemperaturtrennung (18) unterworfen werden.

2. Verfahren (100) nach Anspruch 1, bei dem das zweite Produktgemisch oder dessen der Tieftemperaturtrennung (18) zugeführter Teil und die Leichtgasfraktion oder deren der Tieftemperaturtrennung (18) zugeführter Teil verdichtet (15) und anschließend der Tieftemperaturtrennung (18) unterworfen werden.

3. Verfahren (100) nach einem der vorstehenden Ansprüche, bei dem die Tieftemperaturtrennung eine Demethanisierung umfasst, und bei dem der in dem ersten Einsatzgemisch enthaltene Wasserstoff zumindest zum Teil unter Verwendung der Demethanisierung bereitgestellt wird.

4. Verfahren (100) nach einem der vorstehenden Ansprüche, bei dem unter Verwendung zumindest eines Teils des zweiten Produktgemischs eine Pyrolysebenzinfraktion bereitgestellt wird.

5. Verfahren (100) nach Anspruch 4, bei dem zumindest ein Teil der in dem ersten Einsatzgemisch enthaltenen alkylierten Aromaten durch eine Aufbereitung zumindest eines Teils der Pyrolysebenzinfraktion bereitgestellt wird.

6. Verfahren (100) nach Anspruch 5, bei dem das erste Einsatzgemisch arm an oder frei von nichtaromatischen Kohlenwasserstoffen ist.

7. Verfahren (100) nach Anspruch 6 oder 7, bei dem die Aufbereitung der Pyrolysebenzinfraktion oder deren Teils eine Hydrierung (21) und/oder eine Trennung (22) und/oder eine Hydrodesulfurierung (23) und/oder eine Aromatenextraktion (24) umfasst.

8. Verfahren (100) nach einem der vorstehenden Ansprüche, bei dem der unter Verwendung der Tieftemperaturtrennung (18) bereitgestellte und in dem ersten Einsatzgemisch enthaltene Wasserstoff zur Bildung des ersten Einsatzgemischs einer Verdichtung (31) unterworfen wird.

9. Verfahren (100) nach einem der vorstehenden Ansprüche, bei dem das zweite Einsatzgemisch Naphtha enthält.

10. Verfahren (100) nach einem der vorstehenden Ansprüche, bei dem die alkylierten Aromaten Toluol und Xylole umfassen.

11. Verfahren (100) nach einem der vorstehenden Ansprüche, bei dem zumindest ein Teil des Benzols und der nicht umgesetzten alkylierten Aromaten unter Erhalt einer Flüssigfraktion aus dem ersten Produktgemisch abgetrennt werden.

12. Verfahren (100) nach Anspruch 11, bei dem unter Verwendung zumindest eines Teils der Flüssigfraktion eine überwiegend oder ausschließlich Benzol enthaltende Fraktion und eine überwiegend oder ausschließlich die nicht umgesetzten alkylierten Aromaten enthaltende Fraktion gebildet werden.

13. Verfahren (100) nach Anspruch 12, bei dem die überwiegend oder ausschließlich die nicht umgesetzten alkylierten Aromaten enthaltende Fraktion zumindest zum Teil zu der Hydrodealkylierung zurückgeführt wird.

14. Anlage zur Herstellung von Benzol, mit Mitteln, die dafür eingerichtet sind, ein erstes Einsatzgemisch zu bilden, das alkylierte Aromaten und Wasserstoff enthält, und mit wenigstens einem Reaktor, der dafür eingerichtet ist, die in dem ersten Einsatzgemisch enthaltenen alkylierten Aromaten mit dem in dem ersten Einsatzgemisch enthaltenen Wasserstoff durch Hydrodealkylierung (33) unter Erhalt eines ersten Produktgemischs teilweise zu dem Benzol umzusetzen, wobei das erste Produktgemisch das Benzol, die nicht umgesetzten alkylierte Aromaten, bei der Umsetzung der alkylierten Aromaten zu dem Benzol gebildete Alkane mit einem bis drei Kohlenstoffatomen und den nicht umgesetzten Wasserstoff enthält, und wobei eine oder mehrere Trenneinrichtungen vorgesehen sind, die dafür eingerichtet sind, zumindest ein Teil der Alkane mit einem bis drei Kohlenstoffatomen und des Wasserstoffs unter Erhalt einer Leichtgasfraktion aus dem ersten Produktgemisch abzutrennen, **dadurch gekennzeichnet, dass** Mittel vorgesehen sind, die dafür eingerichtet sind, den in dem ersten Einsatzgemisch enthaltenen Wasserstoff zumindest zum Teil unter Verwendung einer Tieftemperaturtrennung (18) bereitzustellen und dieser zumindest ein Teil eines zweiten Produktgemischs zuzuführen, dass Mittel vorgesehen sind, die dafür eingerichtet sind, das zweite Produktgemisch zumindest zum Teil durch Dampfspalten (11) eines zweiten Einsatzgemischs zu bilden, dass Mittel vorgesehen sind, die dafür eingerichtet sind, die Leichtgasfraktion ebenfalls zumindest zum Teil der Tieftemperaturtrennung (18) zuzuführen, und dass Mittel vorgesehen sind, die dafür eingerichtet sind, das zweite Produktgemisch oder dessen der Tieftemperaturtrennung (18) zugeführten Teil und die Leichtgasfraktion oder deren der Tieftemperaturtrennung (18) zugeführten Teil einer Sauergasentfernung (16) und anschließend der Tieftemperaturtrennung (18) zu unterwerfen.

## Claims

1. Method (100) for the production of benzene, wherein a first feedstock mixture is formed which contains alkylated aromatics and hydrogen, and wherein the alkylated aromatics contained in the first feedstock mixture are partially reacted with the hydrogen contained in the first feedstock mixture to give the benzene through hydrodealkylation (33), to obtain a first product mixture, wherein the first product mixture contains the benzene, the unreacted alkylated aromatics, alkanes with one to three carbon atoms formed in the reaction of the alkylated aromatics to the benzene, and the unreacted hydrogen, and wherein at least part of the alkanes with one to three carbon atoms and of the hydrogen are separated off to obtain a light-gas fraction from the first product mixture, **characterized in that** the hydrogen contained in the first feedstock mixture is provided at least in part using a cryogenic separation (18), to which at least part of a second product mixture is supplied, wherein the second product mixture is formed at least in part through steam cracking (11) of a second feedstock mixture, and **in that** the light-gas fraction is also supplied at least in part to the cryogenic separation (18), wherein the second product mixture or the part thereof supplied to the cryogenic separation (18) and the light-gas fraction or the part thereof supplied to the cryogenic separation (18) are subjected to an acid gas removal (16) and subsequently to the cryogenic separation (18).

2. Method (100) according to claim 1, wherein the second product mixture or the part thereof supplied to the cryogenic separation (18) and the light-gas fraction or the part thereof supplied to the cryogenic separation (18) are compressed (15) and subsequently subjected to the cryogenic separation (18).

3. Method (100) according to one of the preceding claims, wherein the cryogenic separation comprises a demethanization, and wherein the hydrogen contained in the first feedstock mixture is provided at least in part using the demethanization.

4. Method (100) according to one of the preceding claims, wherein a pyrolysis gasoline fraction is provided using at least part of the second product mixture.

5. Method (100) according to claim 4, wherein at least part of the alkylated aromatics contained in the first feedstock mixture is provided by processing at least part of the pyrolysis gasoline fraction.

6. Method (100) according to claim 5, wherein the first feedstock mixture is low in or free of non-aromatic hydrocarbons.

7. Method (100) according to claim 6 or 7, wherein the processing of the pyrolysis gasoline fraction or part thereof comprises a hydrogenation (21) and/or a separation (22) and/or a hydrodesulfurization (23) and/or an aromatics extraction (24).

8. Method (100) according to one of the preceding claims, wherein the hydrogen provided using the cryogenic separation (18) and contained in the first feedstock mixture is subjected to a compression (31) to form the first feedstock mixture.

9. Method (100) according to one of the preceding claims, wherein the second feedstock mixture contains naphtha.

10. Method (100) according to one of the preceding claims, wherein the alkylated aromatics comprise toluene and xylenes.

11. Method (100) according to one of the preceding claims, wherein at least part of the benzene and of the unreacted alkylated aromatics are separated off from the first product mixture to obtain a liquid fraction.

12. Method (100) according to claim 11, wherein, using at least part of the liquid fraction, a fraction containing predominantly or exclusively benzene and a fraction containing predominantly or exclusively the unreacted alkylated aromatics are formed.

13. Method (100) according to claim 12, wherein the fraction containing predominantly or exclusively the unreacted alkylated aromatics is at least partially recycled to the hydrodealkylation.

14. Plant for the production of benzene, having means designed to form a first feedstock mixture which contains alkylated aromatics and hydrogen, and having at least one reactor designed to partially react the alkylated aromatics contained in the first feedstock mixture with the hydrogen contained in the first feedstock mixture to give the benzene through hydrodealkylation (33) to obtain a first product mixture, wherein the first product mixture contains the benzene, the unreacted alkylated aromatics, alkanes with one to three carbon atoms formed in the reaction of the alkylated aromatics to give the benzene, and the unreacted hydrogen, and wherein one or more separators are provided which are designed to separate off at least part of the alkanes with one to three carbon atoms and of the hydrogen, to obtain a light-gas fraction from the first product mixture, **characterized in that** means are provided which are designed to provide the hydrogen contained in the first feedstock mixture at least in part using a cryogenic separation (18), and to supply to same at least part of a second product mixture, **in that** means are provided which are designed to form the second product mixture at least in part through steam cracking (11) of a second feedstock mixture, **in that** means are provided which are designed to also supply the light-gas fraction at least in part to the cryogenic separation (18), and **in that** means are provided which are designed to subject the second product mixture or the part thereof supplied to the cryogenic separation (18) and the light-gas fraction or the part thereof supplied to the cryogenic separation (18) to an acid gas removal (16) and subsequently to the cryogenic separation (18).

## Revendications

1. Procédé (100) de production de benzène, dans lequel un premier mélange de charge est formé, lequel contient des aromatiques alkylés et de l'hydrogène, et dans lequel les aromatiques alkylés contenus dans le premier mélange de charge sont mis en réaction par hydrodésalkylation (33) avec l'hydrogène contenu dans le premier mélange de charge pour obtenir partiellement le benzène en obtenant un premier mélange de produits, le premier mélange de produits contenant le benzène, les aromatiques alkylés n'ayant pas réagi, les alcanes ayant un à trois atomes de carbone formé pendant la réaction des aromatiques alkylés pour obtenir le benzène et l'hydrogène n'ayant pas réagi, et au moins une partie des alcanes ayant un à trois atomes de carbone et de l'hydrogène sont séparés du premier mélange de produits pour obtenir une fraction de gaz léger, **caractérisé en ce que** l'hydrogène contenu dans le premier mélange de charge est fourni au moins partiellement en utilisant une séparation cryogénique (18), à laquelle au moins une partie d'un second mélange de produits est acheminée, le second mélange de produits étant au moins partiellement formé par craquage à la vapeur d'eau (11) d'un second mélange de charge, et **que** la fraction de gaz léger est également au moins partiellement acheminée à la séparation cryogénique (18), le second mélange de produits ou sa partie acheminée à la séparation cryogénique (18) et la fraction de gaz léger ou sa partie acheminée à la séparation cryogénique (18) étant soumis à une élimination de gaz acide (16) et ensuite à la séparation cryogénique (18).

2. Procédé (100) selon la revendication 1, dans lequel le second mélange de produits ou sa partie acheminée à la séparation cryogénique (18) et la fraction de gaz léger ou sa partie acheminée à la séparation cryogénique (18) sont comprimés (15) et ensuite soumis à la séparation cryogénique (18).

3. Procédé (100) selon l'une quelconque des revendications précédentes, dans lequel la séparation cryogénique comprend la déméthanisation, et dans lequel l'hydrogène contenu dans le premier mélange de charge est fourni au moins partiellement en utilisant la déméthanisation.

4. Procédé (100) selon l'une quelconque des revendications précédentes, dans lequel une fraction d'essence de pyrolyse est fournie en utilisant au moins une partie du second mélange de produits.

5. Procédé (100) selon la revendication 4, dans lequel au moins une partie des aromatiques alkylés contenus dans le premier mélange de charge sont fournis par traitement d'au moins une partie de la fraction d'essence de pyrolyse.

6. Procédé (100) selon la revendication 5, dans lequel le premier mélange de charge est pauvre en ou exempt d'hydrocarbures non aromatiques.

7. Procédé (100) selon la revendication 6 ou 7, dans lequel le traitement de la fraction d'essence de pyrolyse ou d'une partie de celle-ci comprend l'hydrogénation (21) et/ou la séparation (22) et/ou l'hydrodésulfuration (23) et/ou l'extraction d'aromatiques (24).

8. Procédé (100) selon l'une quelconque des revendications précédentes, dans lequel l'hydrogène fourni par séparation cryogénique (18) et contenu dans le premier mélange de charge est soumis, pour former le premier mélange de charge, à une compression (31).

9. Procédé (100) selon l'une quelconque des revendications précédentes, dans lequel le second mélange de charge comprend du naphta.

10. Procédé (100) selon l'une quelconque des revendications précédentes, dans lequel les aromatiques alkylés comprennent du toluène et des xylènes.

11. Procédé (100) selon l'une quelconque des revendications précédentes, dans lequel au moins une partie du benzène et des composés aromatiques alkylés n'ayant pas réagi sont séparés du premier mélange de produits pour obtenir une fraction liquide.

12. Procédé (100) selon la revendication 11, dans lequel, en utilisant au moins une partie de la fraction liquide, une fraction contenant majoritairement ou exclusivement du benzène et une fraction contenant majoritairement ou exclusivement les aromatiques alkylés n'ayant pas réagi sont formées.

13. Procédé (100) selon la revendication 12, dans lequel la fraction contenant majoritairement ou exclusivement les aromatiques alkylés n'ayant pas réagi est au moins partiellement réacheminée à l'hydrodésalkylation.

14. Installation de production de benzène, comprenant des moyens, lesquels sont conçus pour former un premier mélange de charge, lequel contient des aromatiques alkylés et de l'hydrogène, et au moins un réacteur, lequel est conçu pour mettre en réaction par hydrodésalkylation (33) les aromatiques alkylés contenus dans le premier mélange de charge avec l'hydrogène contenu dans le premier mélange de charge pour obtenir partiellement le benzène en obtenant un premier mélange de produits, le premier mélange de produits contenant le benzène, les aromatiques alkylés n'ayant pas réagi, les alcanes ayant un à trois atomes de carbone formé pendant la réaction des aromatiques alkylés pour obtenir le benzène et l'hydrogène n'ayant pas réagi, et au moins un ou plusieurs dispositifs de séparation étant prévus, lesquels sont conçu pour séparer au moins une partie des alcanes ayant un à trois atomes de carbone et de l'hydrogène du premier mélange de produits pour obtenir une fraction de gaz léger, **caractérisée en ce que** des moyens sont prévus, lesquels sont conçus pour fournir l'hydrogène contenu dans le premier mélange de charge au moins partiellement en utilisant une séparation cryogénique (18) et pour acheminer à celle-ci au moins une partie d'un second mélange de produits, **que** des moyens sont prévus, lesquels sont conçus pour former le second mélange de produits au moins partiellement par craquage à la vapeur d'eau (11) d'un second mélange de charge, **que** des moyens sont prévus, lesquels sont conçus pour acheminer également au moins partiellement la fraction de gaz léger à la séparation cryogénique (18), et **que** des moyens sont prévus, lesquels sont conçus pour soumettre le second mélange de produits ou sa partie acheminée à la séparation cryogénique (18) et la fraction de gaz léger ou sa partie acheminée à la séparation cryogénique (18) à une élimination de gaz acide (16) et ensuite à la séparation cryogénique (18).
